# EUROPÄISCHE PATENTSCHRIFT

(11) **EP 0 931 543 B1**
(45) Veröffentlichungstag und Bekanntmachung des Hinweises auf die Patenterteilung: **20.03.2002**
(21) Anmeldenummer: 97122492.8
(22) Anmeldetag: 19.12.1997
(51) Int. Cl.: A61K 9/28, A61K 9/20, A61K 35/74

(54) **Mehrschichttablette enthaltend probiotische Mikroorganismen wie z.B. Lactobacilli oder Bifidobakterien**
Multilayered tablet comprising probiotic microorganisms such as lactobacilli or bifidobacteria
Comprimés multicouchés contenant des microorganismes probiotiques comme lactobacilli ou bifidobacteria

(43) Veröffentlichungstag der Anmeldung: 28.07.1999
(73) Patentinhaber: MERCK PATENT GmbH, 64293 Darmstadt (DE)
(72) Erfinder: Fusca, Martino, 55299 Nackenheim (DE); Färber, Dagmar, 64646 Heppenheim (DE)

(56) Entgegenhaltungen:
- PATENT ABSTRACTS OF JAPAN vol. 017, no. 617 (C-1129), 15.November 1993 & JP 05 186337 A (KANEBO LTD), 27.Juli 1993,
- PATENT ABSTRACTS OF JAPAN vol. 096, no. 010, 31.Oktober 1996 & JP 08 143463 A (TANABE SEIYAKU CO LTD), 4.Juni 1996,

## Beschreibung

Die Erfindung betrifft Mehrschichttabletten, die aus zwei, drei oder mehr Schichten aufgebaut sind, wobei eine Schicht probiotische Mikroorganismen enthält, während die anderen Schichten ernährungsphysiologisch wertvolle Lebensmittelinhaltsstoffe, wie Vitamine, Mineralstoffe, etc., enthalten. Die Tabletten zeichnen sich durch eine in Bezug auf die verwendeten Mikroorganismen hohe Stabilität bzw. Wirksamkeit über einen großen Zeitraum aus und sind somit bestens für Lagerhaltung geeignet.

Viele Menschen, insbesondere in den wirtschaftlich und technisch hochentwickelten Ländern, klagen oft über temporäre oder chronische Verdauungsbeschwerden, verursacht durch eine geschädigte oder gestörte Darmflora. Ursache für diese "Wohlstandserkrankungen" sind meist Streßsituationen, Medikamenten- oder Drogenmißbrauch, Folgeerscheinung von Antibiotika-Behandlungen oder auch sehr oft Fehlernährung.
Akute und drastische Beschwerden lassen sich mit bekannten Arzneimitteln, die nicht nur geeignete pharmazeutische Wirkstoffe sondern auch entsprechende natürliche Enzyme oder darmspezifische Mikroorganismen beinhalten können, behandeln.

Bei ständigen, leichten, nicht direkt als Krankheit zu bezeichnenden Störungen des Intestinaltraktes ist hingegen oft schon der regelmäßige Verzehr von geeigneten, speziell ausgesuchten Lebensmitteln oder Nahrungsergänzungspräparaten auf Basis von Mikroorganismen ausreichend, um die Symptome, die eine gestörte oder geschädigte Darmflora hervorrufen, zu lindern oder zu beseitigen. Aber auch bei intakter bzw. gesunder Darmflora kann die Zuführung von probiotischen Mikroorganismen, insbesondere in Verbindung mit Antioxidantien zu immunstimulierender Wirkung führen.

Joghurt- und Sauermilchprodukte erfreuen sich auch aus diesen Gründen einer zunehmenderen Beliebtheit. Die meisten dieser für die Ernährung wertvollen Produkte, die geeignete Mikroorganismen-Kulturen für den genannten Zweck enthalten, sind jedoch Frischwaren und lassen sich somit nur unter Kühlung und dann auch nur wenige Tage aufbewahren.

Produkte, die die entsprechenden Mikroorganismen als Monopräparat offerieren, sind in vielen Ländern nicht als Lebensmittel oder Lebensmittelergänzung zulässig, weil sie keine ernährungsphysiologisch wertvollen Stoffe wie Mineralien, Fette, Vitamine, Kohlenhydrate, Eiweißstoffe, Ballaststoffe oder Spurenelemente, wie sie in Lebensmitteln eben in ausreichender Menge vorliegen, enthalten.

Zudem wurde festgestellt, daß Trockenpräparate auf Basis von die Darmflora positiv stimulierenden, probiotischen Mikroorganismen, insbesondere wenn sie Lebensmittelzusätze der oben genannten Art enthalten, äußerst instabil in Bezug auf ihren Gehalt an biologisch wirksamen Mikroorganismen sind. Selbst in Produkten, die ausschließlich entsprechende Kulturen ohne solche Zusätze enthalten, mußten nach nur kurzen Zeiten teilweise dramatische Verluste an lebenden Mikroorganismen festgestellt werden ("Probiotics, the Friendly Bacteria"; Health Which?, 1997, 134).

Es bestand somit die Aufgabe, Präparate im Lebensmittelbereich, insbesondere für den ständig wachsenden Markt "Functional Food", zur Verfügung zu stellen, welche einerseits eine ausreichende Menge an ernährungsphysiologisch wertvollen Lebensmittelinhaltsstoffen, wie Vitamine, Mineralien, Balaststoffe, etc. aufweisen, andererseits aber eine trotz dieser Zusätze stabil bleibende große Anzahl von probiotisch wirkenden, die Darmflora stabilisierenden Mikroorganismen enthalten.

Diese Aufgabe wurde durch die Bereitstellung einer Mehrschichttablette für den Lebensmittelbereich gelöst. Dabei werden die probiotischen Mikroorganismen in einer getrennten Schicht zur Verfügung gestellt. Die anderen Lebensmittelzusatzstoffe liegen in einer weiteren oder mehreren anderen Schichten vor. Überraschenderweise ist die Stabilität der probiotischen Mikroorganismen in den erfindungsgemäßen Tabletten oftmals sogar höher als in den bekannten, lebensmittelrechtlich nicht zulässigen Präparaten, die ausschließlich probiotische Kulturen enthalten.

Gegenstand der Erfindung ist somit eine Mehrschichttablette für den Lebensmittelbereich, die innerhalb eines Trägermaterials ernährungsphysiologisch wertvolle Substanzen enthält, und die dadurch gekennzeichnet ist, daß sie mindestens aus zwei separaten Schichten besteht, wobei in einer Schicht, als ernährungsphysiologisch wertvolle Substanz, ausschließlich probiotische Mikroorganismen vorliegen.

Besonders vorteilhaft sind Schichttabletten mit drei Schichten. Es hat sich nämlich herausgestellt, daß auch in Bezug auf die Stabiltität der anderen Zusätze, welche verschiedenartige, mehr oder weniger empfindliche und sich gegenseitig beeinflussende, bzw. störende Stoffe sein können, wie beispielsweise Vitamine (z. B. β-Carotin, Vitamin C, Vitamin B-Komplex), Eiweißstoffe, reduzierend wirkende Zuckeralkohole (z. B. Sorbit, Mannit), oxidationsempfindliche Enzyme oder Co-Enzyme oder aber auch physiologisch wirksame Pflanzenextrakte, gute Ergebnisse mit Mehrschichttabletten erhalten werden. Mittels des mehrschichtigen Aufbaus der erfindungsgemäßen Tabletten kann also nicht nur die Stabilität und Lagerfähigkeit der verwendeten Mikroorganismen deutlich gesteigert werden, sondern auch die der anderen im Zusammenspiel miteinander zu Instabilität neigenden Stoffe. Gegenstand der Erfindung ist somit eine entsprechende Dreischichttablette. Aber auch Tabletten mit drei oder mehr Schichten, also z. B. drei, vier oder fünf Schichten sind möglich.
Voraussetzung für eine gute Stabilität in Bezug auf die Anzahl lebender und aktiver Mikroorgansimen in der erfindungsgemäßen Tablette ist neben dem mehrschichtigen Aufbau der Tablette (wobei die Mikroorganismen in einer separaten Schicht ohne andere der genannten Lebensmittelzusätze vorliegen), die Notwendigkeit, daß die besagte Schicht, insbesondere das zur Verwendung kommende Trägermaterial, in dem die Mikroorganismen eingebettet sind, einen Wassergehalt von nicht mehr als 0,1 % aufweist. Dies kann dadurch bewerkstelligt werden, daß das Trägermaterial vor der Vermischung mit der Mikroorganismenkultur sehr sorgfältig getrocknet wird (in der Regel bei 100 - 120°C über einen Zeitraum von mindestens 60 Minuten). Gegenstand der Erfindung ist somit eine entsprechende Mehrschichttablette, welche dadurch gekennzeichnet ist, daß der Wassergehalt der probiotischen Schicht maximal 0,1 % beträgt.

Als Trägermaterialien, bzw. Tablettengrundlage für die einzelnen Schichten kommen die für Tabletten und Dragees üblichen, im Stand der Technik wohl bekannten Stoffe zum Einsatz, also z. B. folgende Verbindungen, sofern sie je nach Land, in dem die Tabletten zum Einsatz kommen, lebensmittelrechtlich zulässig sind: Stärke (z. B. Maisstärke), Talkum, mikrokristalline Cellulose, Lactose, hochdisperses Siliciumdioxid, Polyvinylpyrrolidon oder Cellulosepulver. Als weitere Bestandteile der Tablettengrundlage (z.B. Binde- und Trennmittel) können beispielsweise auch Kohlenhydrate, wie Mannit, Sorbit, Xylit, Glucose, Sucrose, Fructose, Maltose, Dextrose, Maltodextrin, Kaolin oder Cellulosederivate wie Methylcellulose, Hydroxypropylcellulose oder Hydroxypropylmethylcellulose eingesetzt werden, ebenso wie Calciumcarbonat, Calcium, Magnesium- oder Glycerinstearat sowie ferner Farb- und Aromastoffe. Die anteilmäßige Zusammensetzung dieser Grundlagenstoffe richtet sich einerseits natürlich nach dem angestrebten Gehalt an eigentlichen Wirksubstanzen, wie Mikroorganismen, Vitamine, Enzyme, Ballaststoffe usw., und andererseits nach Kriterien, die die mechanisch-physikalischen Eigenschaften der Tablette bzw. ihrer Schichten bestimmen, wie z. B. Härte, Verpressbarkeit, Größe, Form usw.

In den meisten Fällen liegt der Anteil der genannten Träger- und Hilfsstoffe (z. B. Gleit- und Trennmittel, Aromastoffe, Farbstoffe, Geschmacksstoffe, Stabilisatoren, Antoxidantien) zwischen 1 und 80 % bezogen auf die gesamte Tablette, welche erfindungsgemäß eine Masse zwischen 0,5 und 1,5 g, vorzugsweise etwa ein Gramm aufweist. Der Anteil der Hilfsstoffe beträgt 1 bis 20 %.

Die Menge der Mikroorganismen, die bei der Tablettierung eingesetzt werden, sollten erfindungsgemäß zwischen 10⁸ und 10¹⁰ aktive Mikroorganismen pro Tablette betragen. Ein niedrigerer Gehalt an Kulturen gewährleistet nicht mehr den angestrebten Effekt auf die Darmflora. Der Aufbau und die Herstellungsweise der erfindungsgemäßen Tabletten stellen sicher, daß kaum Verluste an lebenden Kulturen auch während eines langen Zeitraums zu beobachten sind. So können bei Einsatz von 10¹⁰ Kulturen bei Anfertigung der Tabletten nach zwei Jahren Lagerung bei 20° C und maximal 50 % Luftfeuchtigkeit immer noch mehr als 10⁹ aktive Mikroorganismen in den Tabletten nachgewiesen werden, unabhängig davon, welche anderen ernärungsphysiologisch wertvollen Stoffe in den benachbarten Schichten vorliegen. Bisherige im Handel befindliche Präparate, welche nicht auf einem Mehrschichtaufbau in Tablettenform beruhen, weisen zum Teil dramatische Verluste von lebenden Zellen auf (Faktor 10 bis 1000).

Gegenstand der Erfindung ist somit eine Mehrschichttablette, welche mindestens 10⁸ probiotische Mikroorganismen in einer Schicht enthält. Gegenstand der Erfindung ist insbesondere eine entsprechende Mehrschichttablette, die dadurch gekennzeichnet ist, daß die probiotische Schicht über einen Lagerungszeitraum von mindestens zwei Jahren unter für Tabletten üblichen Bedingungen und ohne Kühlung mindestens 10⁸ wirksame probiotische Mikroorganismen enthält. Damit ist sichergestellt, daß ohne besondere Lagerhaltungsbedingungen (inbesondere Kühlung), die Tabletten in ihren Verpackungen für eine im Handel üblichen vorgesehenen durchschnittlichen Lagerdauer von einem halben bis einem Jahr in den Regalen der Apotheken oder Läden ohne Wirkungsverlust aufbewahrt werden können.

Als Mikroorganismen, welche in den erfindungsgemäßen Tabletten vorliegen können, sind prinzipiell alle sogenannten probiotischen Kulturen geeignet. Als probiotisch werden erfindungsgemäß alle solche Mikroorganismen bezeichen, die in der gesunden Darmflora entweder selbst üblicherweise vorhanden sind oder einen positven Einfluß auf den gesunden, gestörten oder erkrankten Intestinaltrakt ausüben. Solche Mikroorganismen sind hinreichend bekannt und in den meisten Fällen sind entsprechende Stämme solcher Kulturen für den Lebensmittelbereich auch käuflich erwerbbar. Als geeignete Mikroorganismen im Sinne der Erfindung kommen vor allem Bakterien und Hefen in Frage. Beispiele hierfür sind, ohne die Erfindung dadurch einzuschränken: *Bifidobakterium bifidum, Bifidobakterium longum, Lactobacillus casei, Lactobacillus acidophilus, Saccharomyces thermophilus und Saccharomyces boulardii.* Selbstverständlich können auch Mischungen verschiedenartiger Mikroorganismen in einer Schicht eingesetzt werden.

Zu den ernährungsphysiologisch wertvollen Stoffen, die in den erfindungsgemäßen Tabletten in gemeinsamen oder voneinander getrennten Schichten vorliegen können, zählen erfindungsgemäß insbesondere Vitamine wie Vitamin A (β-Carotin), Vitamin C, Vitamin E, Vitamine des B-Komplexes oder Vitamin K. Die sogenannten ACE-Vitamine sind hierbei von besonderem Interesse. Hierbei können die Vitamine einzeln oder im Gemisch vorliegen. Zuweilen empfiehlt es sich wasserlösliche und fettlösliche (z. B. β-Carotin, Vitamin B12) Vitamine in getrennten Schichten zur Verfügung zu stellen. Die Menge an Vitamin pro Tablette richtet sich in der Regel an der täglichen Mindestbedarfsdosis für das jeweilige Vitamin, die um durchschnittlich 50 - 200 % überschritten werden kann. Für Vitamin C sind dies etwa 100 bis 300 mg, für Vitamin E 10 bis 50 mg, für β-Carotin bis 15 mg und für Vitamin B-Komplex 40 bis 70 mg.

Gegenstand der Erfindung ist somit insbesondere eine Mehrschichttablette für den Lebensmittelbereich, die innerhalb eines Trägermaterials ernährungsphysiologisch wertvolle Substanzen enthält, die dadurch gekennzeichnet ist, daß sie aus zwei Schichten besteht, wobei die erste Schicht, als ernährungsphysiologisch wertvolle Substanz, ausschließlich mindestens 10⁸ probiotische Mikroorganismen und die zweite Schicht Vitamin C oder eine Mischung aus den Vitaminen A (β-Carotin) und C oder eine Mischung aus den Vitaminen A (β-Carotin), C und E enthält.

Gegenstand der Erfindung ist auch eine Mehrschichttablette für den Lebensmittelbereich, enthaltend innerhalb eines Trägermaterials ernährungsphysiologisch wertvolle Substanzen, die dadurch gekennzeichnet ist, daß sie aus drei Schichten besteht, wobei eine äußere Schicht, als ernährungsphysiologisch wertvolle Substanz, ausschließlich mindestens 10⁸ probiotische Mikroorganismen, die zweite Schicht Vitamin C oder eine Mischung aus den Vitaminen A (β-Carotin) und C oder eine Mischung aus den Vitaminen A (β-Carotin), C und E, und die dritte Schicht mindestens ein Vitamin aus dem Vitamin B-Komplex enthält.

Ferner können natürliche Ballaststoffe in den erfindungsgemäßen Tabletten innerhalb bestimmter Schichten zugegen sein. Als natürliche Ballaststoffe sind exemplarisch feingemahlene Soja-, Mais-, oder Weizenkleie oder auch Getreideschrot zu nennen. Bevorzugt wird aufgrund geschmacklicher und farblicher Kriterien Sojakleie. Der Gehalt an Ballaststoffen variiert erfindungsgemäß zwischen 2 und 50 % pro Tablette, je nach dem aus wievielen Schichten die Tablette zusammengesetzt ist.

Weiterhin können einzelne Schichten auch Mineralsalze zwischen 5 und 45 % bezogen auf die gesamte Tablette enthalten. Als Mineralsalze, die einen Einfluß auf den menschlichen lonenhaushalt ausüben, kommen beispielsweise für den Verzehr geeignete anorganische oder organische Natrium-, Kalium-, Calcium, Magnesium- oder Eisensalze in Frage, z. B. Carbonate, Bicarbonate, Phosphate, Biphosphate, Sulfate, Bisulfate, Chloride, Fluoride, Citrate oder Lactate. Ferner können in solchen Schichten auch Spurenelemente wie z. B. Selen vorhanden sein.

Zusätzlich können in vorzugsweise getrennten Schichten auch Magen- und Darmfunktionen fördernde Enzyme, sowie oftmals gegenüber Oxidation und Feuchtigkeit empfindliche Enzyme oder Co-Enzyme (z. B. Co-Enzym Q), die förderlich für den Stoffwechsel oder andere Funktionen im menschlichen Organismus sind, in an sich bekannten Mengen eingebracht werden. Ferner können auch probiotische Substanzen, wie Inulin oder Oligo-Fructose oder anderer Oligo-Zucker, in den verschiedenen Schichten der erfindungsgemäßen Tablette vorliegen. Schließlich kann auch vorgesehen werden, daß einzelne Schichten Pflanzenextrakte, wie beispielsweise Trockenextrakte aus Echinaceae, enthalten.

Die Herstellung der erfindungsgemäßen Tabletten kann in zweierlei Weise erfolgen:
Die trockenen bzw. vorgetrockeneten Pulvermischungen aus Träger- und Hilfsstoffen und Wirkstoffen (Mikroorganismen, Vitamine, Eiweißstoffe etc.) werden in einer handelsüblichen Tablettiermaschine in zwei, drei oder mehr Schichten vorsichtig übereinander geschichtet. Dabei muß nochmals betont werden, daß die Schicht, welche die Mikroorganismen enthält, keine weiteren (diese destabilisierenden) Wirkstoffe enthalten darf, sondern lediglich noch Träger- und Hilfsstoffe und ggf. inerte Wirkstoffe, die nicht zur Vernichtung von lebenden Zellen beitragen, aufweisen sollte. Diese Trägerstoffe müssen erfindungsgemäß sehr gut vorgetrocknet sein und einen Wassergehalt von max. 0,1 % aufweisen. Aus verfahrenstechnischen Gründen kann es vorteilhaft sein, daß die Schicht, welche die Mikroorganismen enthält, eine Außenschicht ist, jedoch kann diese auch bei Tabletten mit mehr als zwei Schichten eine innere Schicht darstellen. Die so übereinandergelagerten Pulverschichten werden nun endgültig zu einer Mehrschichttablette tablettiert. Der Preßdruck wird zwischen 50 und 120 Newton gewählt.

Alternativ zu dieser Tablettierungsmethode können die einzelnen Schichten separat für sich vorgepresst werden, wobei Preßdrucke zwischen 20 und 80 Newton die Regel sind. Die so vorgepreßten Schichten werden anschließend bei Preßdrucken zwischen 50 und 120 Newton zur fertigen Mehrschichttablette gepreßt. Diese Preßmethode hat den Vorteil, daß man die Schichten, welche aufgrund ihrer unterschiedlichen Zusammensetzung eine unterschiedliche Verpreßbarkeit besitzen, individuellen Preßdrucken aussetzen kann, was sowohl hinsichtlich der Haltbarkeit der Mehrschichtablette als Ganzes als auch hinsichtlich der Stabilität der Wirkstoffe in den Einzelschichten vorteilhaft sein kann. Ferner besitzt die Grenzschicht zwischen zwei aneinanderliegenden Schichten durch das Vorpressen eine kleinere aktive Oberfläche, wodurch die Möglichkeit der Reaktion oder Destabilisierung von empfindlichen Wirkstoffen, inklusive der Mikroorganismen, in den beiden Schichten herabgesetzt wird.

Die folgenden Beispiele erläutern die Erfindung.

### Beispiel 1:

In eine handelsüblichen Tablettenpresse wird zunächst eine pulverige erste Schicht bestehend aus Maisstärke, probiotischen Bakterien von Lactobacillus acidophilus und Glycerinstearat und darüber eine zweite Schicht aus Maisstärke und einem Gemisch aus den Vitaminen β-Carotin, C und E eingebracht. Die erste Schicht enthält zwischen 10⁸ und 10¹⁰ Bakterien in 500 mg Maisstärke (30 mg Glycerinstearat). Die zweite Schicht enthält 100 mg Vitamin C, 40 mg Vitamin E und 10 mg β-Carotin vermischt mit 250 mg Maisstärke (insgesamt 900 mg). Die verwendete Maisstärke wurde zuvor bei 105°C für 90 Minuten getrocknet und weist danach einen Wassergehalt von 0.075 % auf. Die Schichten werden in einem Arbeitsgang mit 80 Newton Preßdruck zu einer zweischichtigen Tablette verpreßt.

### Beispiel 2:

Analog Beispiel 1 wird eine Zweischichttablette hergestellt, welche in der zweiten Schicht 150 mg Vitamin C enthält.

### Beispiel 3:

Analog Beispiel 1 wird eine Zweischichttablette hergestellt, deren zweite Schicht anstelle der Vitamine 200 mg feingemahlene Sojakleie und 50 mg Mineralsalzmischung enthält.

### Beispiel 4:

In eine handelsüblichen Tablettenpresse wird zunächst eine pulverige erste Schicht bestehend aus Maisstärke, probiotischen Bakterien von Bifidobakterium bifidum und Glycerinstearat als Trennmittel, darüber eine zweite Schicht aus Maisstärke (+ Trennmittel) und einem Gemisch aus den Vitaminen β-Carotin, C und E, und eine dritte Schicht aus Maisstärke (+ Trennmittel und Vitamin B-Komplex) eingebracht. Die erste Schicht enthält zwischen 10⁸ und 10¹⁰ Bakterien in 500 g Maisstärke. Die zweite Schicht enthält 100 mg Vitamin C, 40 mg Vitamin E und 10 mg β-Carotin vermischt mit 250 mg Maisstärke, und die dritte Schicht enthält 10 mg Vitamin B-Komplex in 100 mg Maisstärke. Die verwendete Maisstärke wurde zuvor bei 105°C für 90 Minuten getrocknet und weist danach einen Wassergehalt von 0.075 % auf. Die Schichten werden in einem Arbeitsgang mit 110 Newton Preßdruck zu einer dreischichtigen Tablette verpreßt.

### Beispiel 5:

Analog Beispiel 4 wird eine dreischichtige Tablette hergestellt, welche in der dritten Schicht, anstelle des Vitamin B-Komplexes, eine Mischung aus Mineralsalzen (250 mg) enhält.

### Beispiel 6:

Analog Beispiel 4 wird eine dreischichtige Tablette hergestellt, wobei die mittlere Schicht die probiotischen Mikroorganismen enthält.

### Beispiel 7:

Es wird analog Beispiel 1 eine zweischichtige Tablette hergestellt. Die beiden Schichten werden jedoch einzeln vorgepreßt (Preßdruck 40 Newton). Anschließend erfolgt die Verpressung zu einer einzigen zweischichtigen Tablette (Preßdruck 90 Newton).

### Beispiel 8:

Es wird analog Beispiel 4 eine dreischichtige Tablette hergestellt. Die beiden Schichten werden jedoch einzeln vorgepreßt (Preßdruck 40 Newton). Anschließend erfolgt die Verpressung zu einer einzigen dreischichtigen Tablette (Preßdruck 100 Newton).

### Beispiel 9:

Es wird analog Beispiel 1 eine zweischichtige Tablette hergestellt, wobei die zweite Schicht anstelle der Vitamine 400 mg eines Echinaceae Trockenextraktes enthält.

## Patentansprüche

1. Mehrschichttablette für den Lebensmittelbereich, die innerhalb eines Trägermaterials ernährungsphysiologisch wertvolle Substanzen enthält, **dadurch gekennzeichnet, daß** sie mindestens aus zwei separaten Schichten besteht, wobei in einer Schicht, als ernährungsphysiologisch wertvolle Substanz, ausschließlich probiotische Mikroorganismen vorliegen.

2. Mehrschichtablette nach Anspruch 1, **dadurch gekennzeichnet, daß** sie aus drei Schichten besteht.

3. Mehrschichttablette nach einem der Ansprüche 1 oder 2, **dadurch gekennzeichnet, daß** der Wassergehalt der probiotischen Schicht maximal 0,1 % beträgt.

4. Mehrschichttablette nach einem der Ansprüche 1 bis 3, **dadurch gekennzeichnet, daß** die anderen Schichten ernährungsrelevante Zusätze, ausgewählt aus den Vitaminen, Mineralstoffen, Spurenelementen, Ballaststoffen, Enzymen und Fetten, enthalten.

5. Mehrschichttablette nach einem der Ansprüche 1 bis 4, **dadurch gekennzeichnet, daß** die probiotische Schicht mindestens 10⁸ probiotische Mikroorganismen enthält.

6. Mehrschichttablette nach Anspruch 5, **dadurch gekennzeichnet, daß** die probiotische Schicht über einen Lagerungszeitraum von mindestens zwei Jahren unter für Tabletten üblichen Bedingungen und ohne Kühlung mindestens 10⁸ ernährungspysiologisch wirksame probiotische Mikroorganismen enthält.

7. Mehrschichttablette nach einem der Ansprüche 1 bis 6, **dadurch gekennzeichnet, daß** sie probiotische Mikroorganismen enthält, welche ausgewählt sind aus der Gruppe: Lactobacillus casei, Lactobacillus acidophilus, Bifidobakterium bifidum, Bifidobakterium longum oder Saccharomyces boulardii.

8. Mehrschichttablette für den Lebensmittelbereich, die innerhalb eines Trägermaterials ernährungsphysiologisch wertvolle Substanzen enthält, **dadurch gekennzeichnet, daß** sie aus zwei Schichten besteht, wobei die erste Schicht, als ernährungsphysiologisch wertvolle Substanz, ausschließlich mindestens 10⁸ probiotische Mikroorganismen und die zweite Schicht Vitamin C oder eine Mischung aus den Vitaminen β-Carotin und C oder eine Mischung aus den Vitaminen β-Carotin, C und E enthält.

9. Mehrschichttablette für den Lebensmittelbereich, enthaltend innerhalb eines Trägermaterials ernährungsphysiologisch wertvolle Substanzen, **dadurch gekennzeichnet, daß** sie aus drei Schichten besteht, wobei eine Schicht, als ernährungsphysiologische Substanz, ausschließlich mindestens 10⁸ probiotische Mikroorganismen, die zweite Schicht Vitamin C oder eine Mischung aus den Vitaminen β-Carotin und C oder eine Mischung aus den Vitaminen β-Carotin, C und E, und die dritte Schicht mindestens ein Vitamin aus dem Vitamin B-Komplex enthält.

10. Verfahren zur Herstellung einer Mehrschichttablette gemäß einem der Ansprüche 1 bis 9, **dadurch gekennzeichnet, daß** man die entsprechenden Mischungen, die die Schichten bilden sollen, schichtenweise abfüllt und anschließend gemeinsam verpreßt, oder jede einzelne Schicht vorpreßt und die vorgepreßten Schichten zu einer einzigen Tablette verpreßt.

11. Verwendung von probiotischen Mikroorganismen zur Herstellung von lagerungsstabilen Mehrschichttabletten gemäß einem der Ansprüche 1-9 für den Lebensmittelbereich.

## Claims

1. Multilayer tablet for the foodstuffs sector, which within a carrier material contains substances valuable in nutritional physiology, **characterized in that** it consists of at least two separate layers, exclusively probiotic micro-organisms being present in one layer as the substance valuable in nutritional physiology.

2. Multilayer tablet according to Claim 1, **characterized in that** it consists of three layers.

3. Multilayer tablet according to either of Claims 1 or 2, **characterized in that** the water content of the probiotic layer is at most 0.1%.

4. Multilayer tablet according to one of Claims 1 to 3, **characterized in that** the other layers contain nutritionally relevant additives, selected from the vitamins, minerals, trace elements, bulkage, enzymes and fats.

5. Multilayer tablet according to one of Claims 1 to 4, **characterized in that** the probiotic layer contains at least 10⁸ probiotic micro-organisms.

6. Multilayer tablet according to Claim 5, **characterized in that** the probiotic layer contains at least 10⁸ probiotic micro-organisms active in nutritional physiology over a storage period of at least two years under conditions customary for tablets and without cooling.

7. Multilayer tablet according to one of Claims 1 to 6, **characterized in that** it contains probiotic micro-organisms which are selected from the group consisting of: Lactobacillus casei, Lactobacillus acidophilus, Bifidobacterium bifidum, Bifidobacterium longum or Saccharomyces boulardii.

8. Multilayer tablet for the foodstuffs sector, which within a carrier material contains substances valuable in nutritional physiology, **characterized in that** it consists of two layers, the first layer exclusively containing at least 10⁸ probiotic micro-organisms as the substance valuable in nutritional physiology and the second layer containing vitamin C or a mixture of the vitamins β-carotene and C or a mixture of the vitamins β-carotene, C and E.

9. Multilayer tablet for the foodstuffs sector, comprising within a carrier material substances valuable in nutritional physiology, **characterized in that** it consists of three layers, one layer exclusively containing at least 10⁸ probiotic micro-organisms as the substance valuable in nutritional physiology, a second layer containing vitamin C or a mixture of the vitamins β-carotene and C or a mixture of the vitamins β-carotene, C and E, and the third layer containing at least one vitamin of the vitamin B complex.

10. Process for the production of a multilayer tablet according to one of Claims 1 to 9, **characterized in that** the corresponding mixtures which are to form the layers are dispensed in layers and then jointly compressed, or each individual layer is preformed and the preformed layers are compressed to give a single tablet.

11. Use of probiotic micro-organisms for the production of storage-stable multilayer tablets according to one of Claims 1-9 for the foodstuffs sector.

## Revendications

1. Comprimé multicouche pour le domaine alimentaire, qui, à l'intérieur d'une matière servant de véhicule, contient des substances de valeur pour la physiologie de la nutrition, **caractérisé en ce qu'**il consiste au moins en deux couches distinctes, exclusivement des micro-organismes probiotiques étant présents, en tant que substance de valeur pour la physiologie de la nutrition, dans une couche.

2. Comprimé multicouche selon la revendication 1, **caractérisé en ce qu'**il consiste en trois couches.

3. Comprimé multicouche selon la revendication 1 ou 2, **caractérisé en ce que** la teneur en eau de la couche probiotique est d'au maximum 0,1 %.

4. Comprimé multicouche selon l'une quelconque des revendications 1 à 3, **caractérisé en ce que** les autres couches contiennent des additifs en rapport avec la nutrition, choisis parmi les vitamines, substances minérales, oligo-éléments, fibres alimentaires, enzymes et matières grasses.

5. Comprimé multicouche selon l'une quelconque des revendications 1 à 4, **caractérisé en ce que** la couche probiotique contient au moins 10⁸ micro-organismes probiotiques.

6. Comprimé multicouche selon la revendication 5, **caractérisé en ce que**, pendant un temps de stockage d'au moins deux ans dans des conditions usuelles pour des comprimés et sans refroidissement, la couche probiotique contient au moins 10⁸ micro-organismes probiotiques actifs pour la physiologie de la nutrition.

7. Comprimé multicouche selon l'une quelconque des revendications 1 à 6, **caractérisé en ce qu'**il contient des micro-organismes probiotiques qui sont choisis dans le groupe : *Lactobacillus casei, Lactobacillus acidophilus, Bifidobacterium bifidum, Bifidobacterium longum* et *Saccharomyces boulardii.*

8. Comprimé multicouche pour le domaine alimentaire, qui, à l'intérieur d'une matière servant de véhicule, contient des substances de valeur pour la physiologie de la nutrition, **caractérisé en ce qu'**il consiste en deux couches, la première couche, en tant que substance de valeur pour la physiologie de la nutrition, contenant exclusivement au moins 10⁸ micro-organismes probiotiques, et la seconde couche contenant de la vitamine C ou un mélange des vitamines β-carotène et C ou un mélange des vitamines β-carotène, C et E.

9. Comprimé multicouche pour le domaine alimentaire, contenant, à l'intérieur d'une matière servant de véhicule, des substances de valeur pour la physiologie de la nutrition, **caractérisé en ce qu'**il consiste en trois couches, une couche, en tant que substance pour la physiologie de la nutrition, contenant exclusivement au moins 10⁸ micro-organismes probiotiques, la deuxième couche contenant de la vitamine C ou un mélange des vitamines β-carotène et C ou un mélange des vitamines β-carotène, C et E, et la troisième couche contenant au moins une vitamine appartenant au complexe des vitamines B.

10. Procédé pour la fabrication d'un comprimé multicouche selon l'une quelconque des revendications 1 à 9, **caractérisé en ce que** les mélanges correspondants, qui sont destinés à former les couches, sont déposés en couches et ensuite pressés ensemble, ou chaque couche individuelle est pressée au préalable et les couches pressées au préalable sont pressées en un comprimé unique.

11. Utilisation de micro-organismes probiotiques, pour la fabrication de comprimés multicouches stables au stockage, selon l'une quelconque des revendications 1 à 9, pour le domaine alimentaire.
